# EUROPEAN PATENT SPECIFICATION

(11) **EP 0 610 078 B1**
(45) Date of publication and mention of the grant of the patent: **16.04.1997**
(21) Application number: 94300780.7
(22) Date of filing: 02.02.1994
(51) Int. Cl.: C07K 7/56, C07K 7/06, A61K 38/00

(54) **Cytotoxic and antiviral compound**
Cytotoxische und antivirale Verbindung
Agent cytotoxique et antiviral

(30) Priority: 03.02.1993 GB 9302046
(43) Date of publication of application: 10.08.1994
(73) Proprietor: PHARMA MAR, S.A., 28760 Tres Cantos, Madrid (ES)
(72) Inventor: Schauer, Paul J., Hawaii (US); Hamann, Mark T., Oxford, MS 38655 (US); Gravalos, Dolores G. c/o Pharma Mar, S.A.,, 28760 Tres Cantos, Madrid (ES)
(74) Representative: Ruffles, Graham Keith

(56) References cited:
- EP-A- 0 358 418
- EP-A- 0 399 685

## Description

An antineoplastic depsipeptide from the Indian Ocean sea have Dolabella auricularia is described in EP-A-399685.

This invention is concerned with a cytotoxic and antiviral compound isolated from the sacoglossan, Elysia rafescens.

According to the present invention there is provided, a new compound, the peptide, Kahalalide F, of the formula:

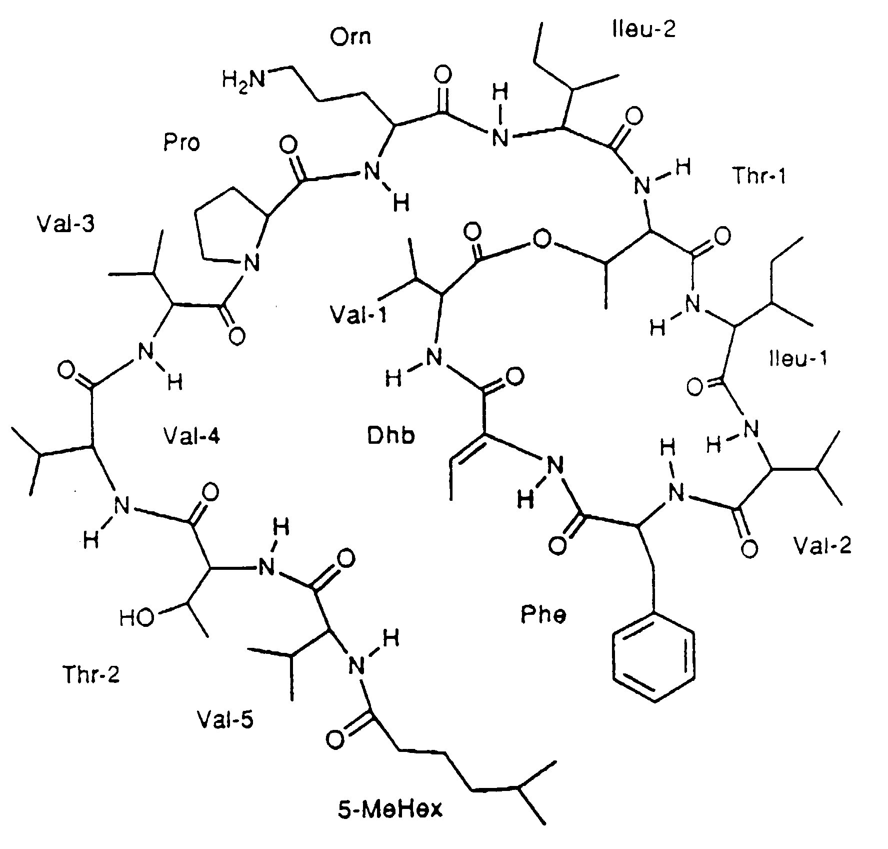

The antitumor activities of this compound has been determinated "in vitro" in cell cultures of human lung carcinoma A-549 and human colon carcinoma HT-29. The procedure was carried out using the methodology descnbed by Raymond J. Bergeron et al. *Biochem. Bioph. Res. Comm. 1984,* 121(3), 848-854 and by Alan C. Schroeder et al. *J. Med, Chem.* 1981, 24 1078-1083.

The antiviral activities of this compound have also been determinated "in vitro" against HSV (Herpes simplex virus) and VSV (Vesicular stomatitis virus). The methodology used to carry out this determination is descnbed by Raymond J. Bergeron et al. *Biochem. Bioph. Res. Comm.* 1984, 121(3), 848-854 and by Alan C. Schroeder et al. *J. Med. Chem.* 1981, 24 1078-1083.

Therefore, the present invention also provides Kahalalide F of use in a method of treating any mammal affected by a malignant tumor sensitive to the compound, which comprises administering to the affected individual a therapeutically effective amount of the compound or a pharmaceutically composition thereof; and of use in a method of treating viral infections in mammals, comprising administering to a patient in need of such treatment, an antiviral effective amount of the compound of the present invention.

The present invention also relates to pharmaceutical preparations which contain as active ingredient the compound, or a pharmaceutical acceptable acid addition salt thereof, as well as the process for its preparation.

Examples of pharmaceutical compositions include any solid (tablets, pills, capsules, granules, etc.) or liquid (solutions, suspensions or emulsions) suitable composition for oral, topical or parenteral administration, and they may contain the pure compound or in combination with any carrier or other pharmacologically active compounds. These compositions may need to be sterile when administered parenterally.

The correct dosage of a pharmaceutical composition of the compound will vary according to the particular formulation, the mode of application and particular situs, host and tumor being treated. Other factors like age, body weight, sex, diet, time of administration, rate of excretion, condition of the host, drug combinations, reaction sensitivities and severity of disease shall be taken in account. Administration can be carried out continuously or periodically within the maximum tolerated dose.

Kahalalide F was isolated from the sacoglossan, Elysia rufescens (family Plakobranchidae, order Sacoglossa), collected near Black point, Oahu. This animal varies in size between 1 and 4 cm; it is dark red-brown in color with light-colored spots. There is orange fringing of the parapodia, which have very small dark green spots from sequestered chloroplasts. Elysia rufescens feeds on the delicate, feather-like green alga *Bryopsis sp.*¹ Kahalalide F can also be isolated from this alga. Two hundred animals were collected over the period of several weeks during spring, 1991 and extracted with EtOH. The extracts were then chromatographed by silica gel flash chromatography (hexane, hexane/EtOAc (1:1), EtOAc, EtOAc (1:1), MeOH and MeOH/HOAc (98:2). The peptides were eluted with EtOAc/MeOH (1:1). Final purification was accomplished by repeated HPLC (RP C18) using MeCN/H₂O with 0.1% TFA (70-45% H₂O) (Figure 1).

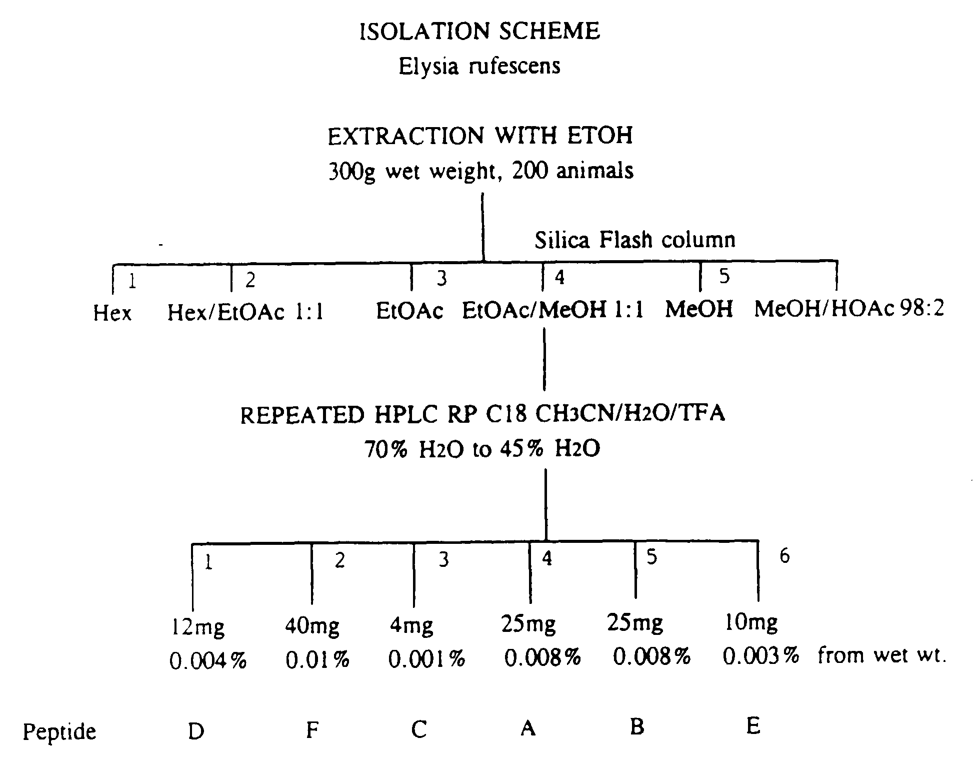

The structures of the peptides were elucidated by 2D NMR experiments (HMQC, HMBC, TOCSY, COSY and ROESY).

Kahalalide F was isolated as a white amorphous powder in 0.02% yield. A molecular formula of C₇₅H₁₂₄N₁₄O₁₆ was deduced from detailed analyses of the ¹³C and ¹H NMR spectra and the high resolution FAB mass spectrum. The 14 substructures in this compound arise from five valines, two isoleucines, two threonines, ornithine, dehydroaminobutyric acid, proline, phenilalanine and 5-methylhexanoic acid (5-MeHex). Kahalalide F is the largest peptide in this series of compounds.

### EXPERIMENTAL

### General Considerations

Optical rotations were measured on a Jasco DIP-370 digital polarimeter. Infrared spectra were recorded on a Nicolet MX-5 FTIR spectrometer. Gas chromatography was accomplished using a Hewlett-Packard Model 5890 instrument. Mass spectra were measured on a VG-70SE magnetic sector mass spectrometer. NMR spectra were measured on a General Electric QE-300 or a GN OMEGA 500 instrument. ¹H NMR chemical shifts are reported in ppm with the chemical shift of the residual protons of the solvent used as internal standards. ¹³C NMR chemical shifts are reported in ppm by using the natural abundance ¹³C of the solvent as an internal standard. Ultraviolet spectra were recorded on a Hewlett-Packard Model 8452A diode array spectrophotometer. All solvents were destilled from glass before use.

Two hundred sacoglossans (Elysia rufescens, Fig. 33) were collected at Black Point, O'ahu during April and May 1992, and extracted 3 times with EtOH. Spring appears to be the time of year Elysia rufescens is in greatest abundance at Black Point. The combined extracts were then chromatographed using silica gel flash chromatography (hexane, hexane/EtOAc (1:1), EtOAc, EtOAc/MeOH (1:1), MeOH, MeOH/HOAc (98:2). The depsipeptides were found in the EtOAc/MeOH (1:1) fraction. Repeated HPLC RP18 MeCN/H₂O/TFA (55/45/1) - MeCN/H₂O/TFA ((30/70/1) yielded six new depsipeptides. For details see Fig. 1.

### KAHALALIDE F

Final purification was accomplished by HPLC on RP18 MeCN/H₂O/TFA (55/45/ 1).Physical data: [α]D-8°(*c* 4.32, MeOH); ¹H NMR (500 MHz, TFA/DMF); amino acid unit, δ (carbon position, mult, J): *Val-1* 4.16 (2, t, J=9.0 Hz), 7.11 (NH on 2, d, J=8.9 Hz), 1.77 (3, m), 0.95 (4, m), 0.95 (5, m); *Dhb* 9.20 (NH on 2, s), 6.48 (3, q, J=6.9 Hz), 1.43 (4, d, J=6.6 Hz); *Phe* 4.68 (2, q, J=6.6 Hz), 8.62 (NH on 2, d, J=6.6 Hz), 3.2 (3, dd, J=13.7, 7.2 Hz), 3.0(3, dd, J=13.7, 9.0 Hz), 7.32 (5, d, J=7.2 Hz), 7.28 (6, t, J=7.5 Hz), 7.21 (7, t, J=7.2 Hz); *Val-2* 4.36 (2, m), 7.82 (NH on 2, d, J=6.6 Hz), 2.12 (3, m), 0.85 (4, m), 0.77 (5, d, J=6.6 Hz); *lleu-1* 4.53 (2,m), 8.38 (NH on 2, d, J=9.6 Hz), 1.98 (3, m), 0.92 (4, d, J=6.6 Hz), 1.40 (5, m), 1.13 (5, m), 0.88 (6, t, J=7.2 Hz); *Thr-1* 4.63 (2, t, J=9.3 Hz), 8.12 (NH on 2, d, J=5.7), 5.07 (3, dq, 9.6, 6.0 Hz), 1.18 (4, d, J=6.3 Hz); *lleu-2* 4.52 (2, m), 7.72 (NH on 2, d, J=8.4 Hz), 1.88 (3, m), 0.88 (4, d, J=6.3 Hz), 1.40 (5, m), 1.13 (5, m), 0.88 (6, d, J=7.2 Hz); *Orn* 4.48 (2, m), 7.92 (NH on 2, d, J=7.8 Hz), 1.76 (3, m), 1.83 (4, m), 3.10 (5, p, J=5.1Hz); *Pro* 4.42 (2, m), 2.12 (3, m), 1.97 (3, m), 2.02 (4, m), 1.88 (4, m), 3.75 (5, m), 3.68 (5, m); *Val-3* 4.41 (2, m), 7.90 (NH on 2, d, J=7.2 Hz), 2.17 (3, m), 0.95 (4, m), 0.85 (5, m); *Val-4* 4.34 (2, m), 7.68 (NH on 2, d, J=8.1 Hz), 2.17 (3, m), 0.95 (4, m), 0.90 (5, m); *Thr-2* 4.46 (2, m), 7.77 (NH on 2, d, J=8.1), 4.21 (3, dq, 6.3, 3.6 Hz), 1.12 (4, d, J=6.6); *Val-5* 4.32 (2, m), 7.85, (NH on 2, d, J=8.1 Hz), 7.82 (NH on (second conformation), d, J=8.1 Hz), 2.14 (3, m), 0.95 (4, m), 0.90 (5, m); 5-*MeHex* 2.26 (2, m), 1.60 (3, m), 1.20 (4, m), 1.55 (5, m), 0.87 (6, d, J=7.2 Hz), 0.87 (7, d, J=7.2 Hz); 5-*MeHex* 2.29 (2,m), 1.65 (3, m), 1.40 (3, m), 1.13 (4, m), 1.35 (5, m), 0.90 (6, m), 0.90 (7, m); ¹³C NMR (125 MHz TFA/DMF): amino acid unit, δ (carbon position); *Val-1* 170.40 (1), 60.31 (2), 30.75 (3), 19.58 (4), 18.76 (5); *Dhb* 164.54 (1), 130.30 (2), 131.26 (3), 12.68 (4); *Phe* 171.31 (1), 56.27 (2), 36.79 (3), 138.23 (4), 129.86 (5), 128.77 (6), 126.98 (7); *Val-2* 172.94 (1), 58.57 (2), 32.38 (3), 18.92 (4), 17.60 (5); *Ileu-1* 171.87 (1), 57.48 (2), 38.78 (3), 14.56 (4), 26.78 (5), 11.67; *Thr-1* 169.68 (1), 57.37 (2), 71.05 (3), 17.34 (4); *Ileu-2* 171.92 (1), 57.29 (2), 38.01 (3), 14.78 (4), 26.55 (5), 11.63 (6); *Orn* 172.01 (1), 52.87 (2), 29.63 (3), 24.39 (4), 40.05 (5); *Pro* 172.55 (1), 60.23 (2), 29.58 (3), 25.38 (4), 48.03 (5); *Val-3* 171.28 (1), 57.57 (2), 30.54(3), 19.61 (4), 18.80 (5); *Val-4* 171.83 (1), 59.10 (2), 31.26 (3), 19.45 (4), 18.08 (5); *Thr-2* 170.97 (1),, 58.89 (2), 67.36 (3), 19.66 (4); *Val-5* 172.67 (1), 59.64 (2), 30.66 (3), 19.61 (4), 18.43 (5); *5-MeHex* 173.83 (1), 36.28 (2), 23.99 (3), 38.96 (4), 28.10 (5), 22.54 (6), 22.50 (7); *5-MeHex* (second conformation) 174.08 (1), 33.86 (2), 32.84 (3), 29.75 (4), 34.54 (5), 19.51 (6), 11.20 (7); IR neat (NaCl): 3287 (s, br), 2964 (s, br), 1646 (s), 1528 (s), 1465 (s), 1388 (m), 1228 (m), cm⁻¹; mass spectrum HRFAB *m/z* (fragment, %) 1477.9408 (M⁺ + 1,85)(calcd for C₇₅H₁₂₅N₁₄O₁₆: 1477.9398); UV (MeOH): λₘₐₓ 204 (89,630)nm.

Amino acid analysis by GC-MS with a Chirasil-Val column indicates that Kahalalide F consists of D-Ileu, -Orn, L-Phe, D-Pro, L-Thr, D-Allo-Thr, 3 D-Val and 2 L-Val.

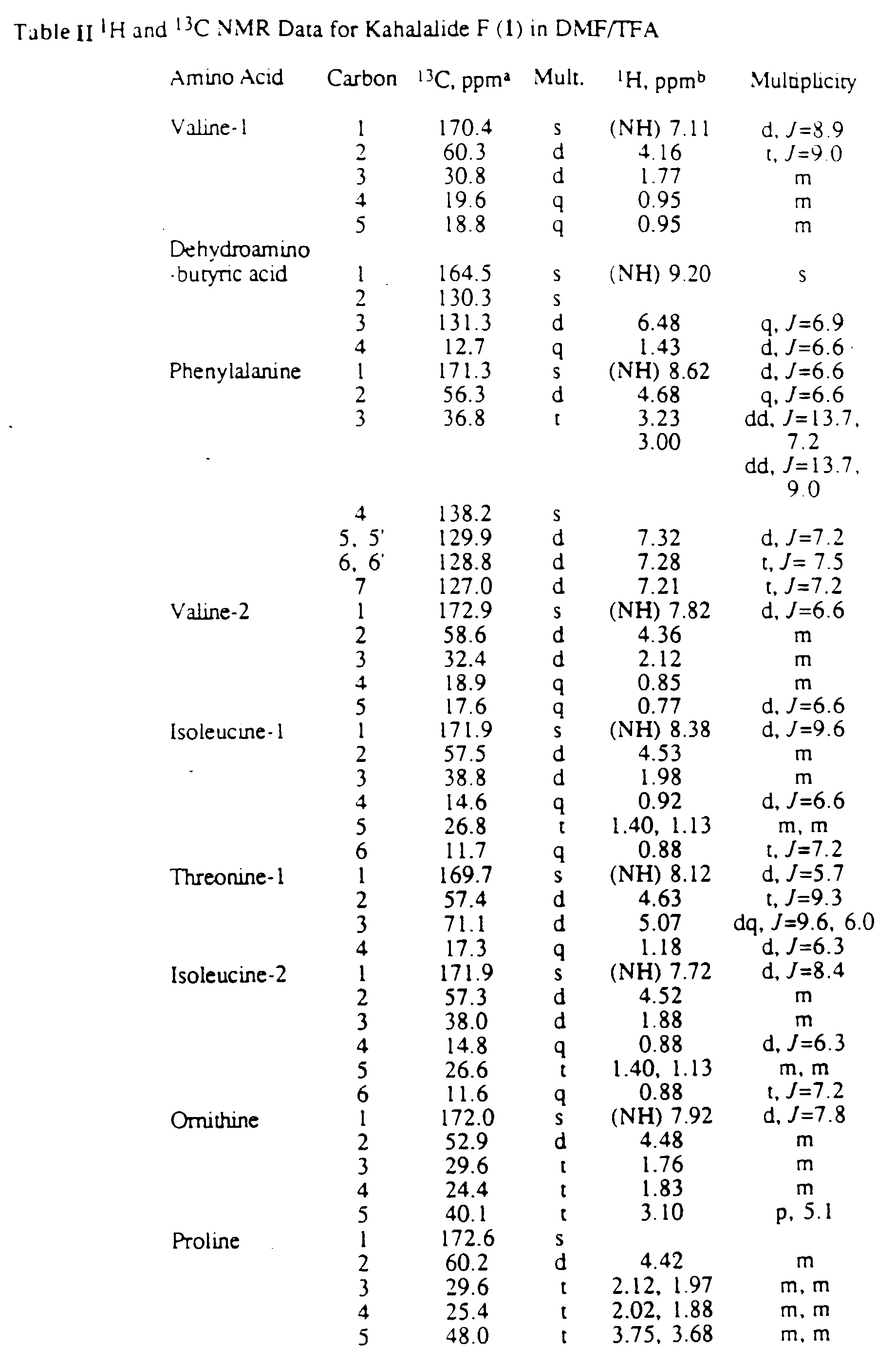

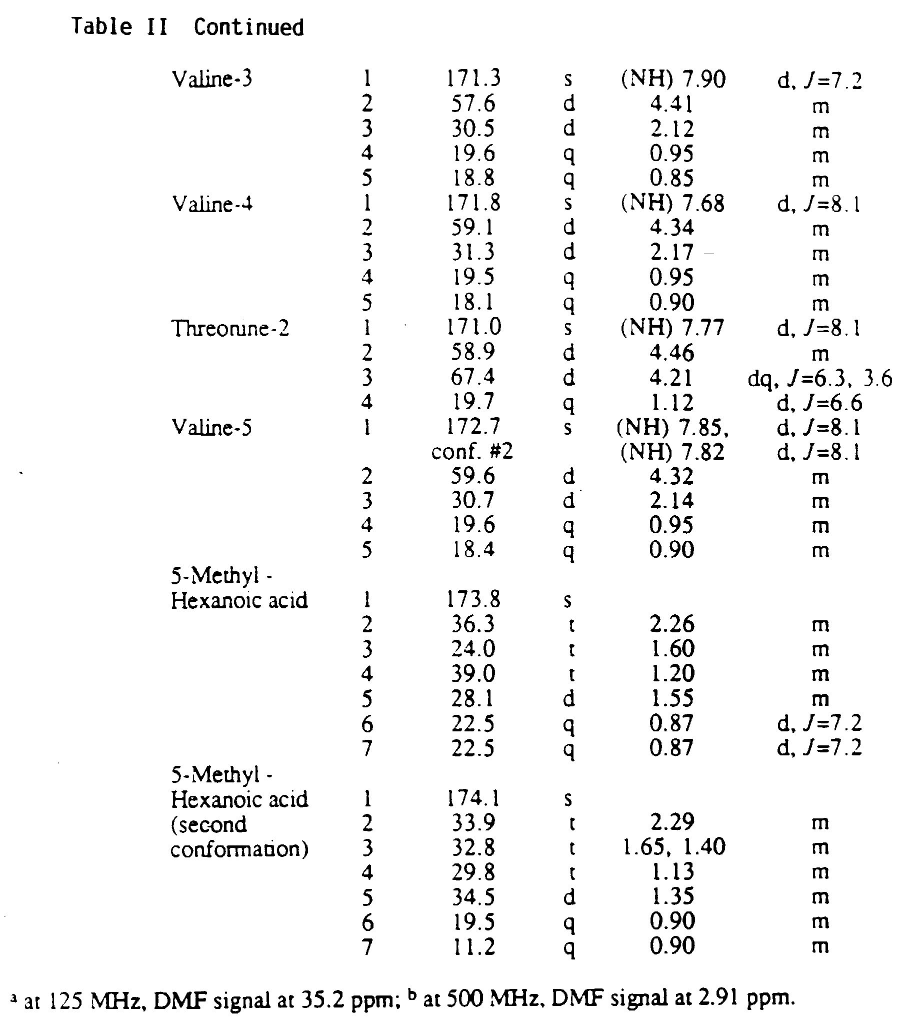

**Table I.**

| *In vitro* Activity of Kahalalide F from *Elysia rufescens* Assay (M.I.C. µg/mL) | |
|---|---|
| Cytotoxicity µg/mL (IC₅₀) | |
| A-549 | 2.5 |
| HT-29 | 0.25-0.5 |
| | |
| Antiviral µg/mL (% reduction) | |
| Mv 1 Lu/HSV II | 0.5 (95%) |
| CV-1/HSV-1 | >8 |
| BHK/VSV | >8 |
| | |
| Antifungal 6mm disk | 50 µg/disk |
| Aspergillus oryzae | 19 mm |
| Penicillium notatum | 26 mm |
| Tricophyton mentagrophy | 34 mm |
| Saccharomyces cerevisiae | neg |
| Candida albicans | 16 mm |

## Claims

1. Kahalalide F of the formula: -

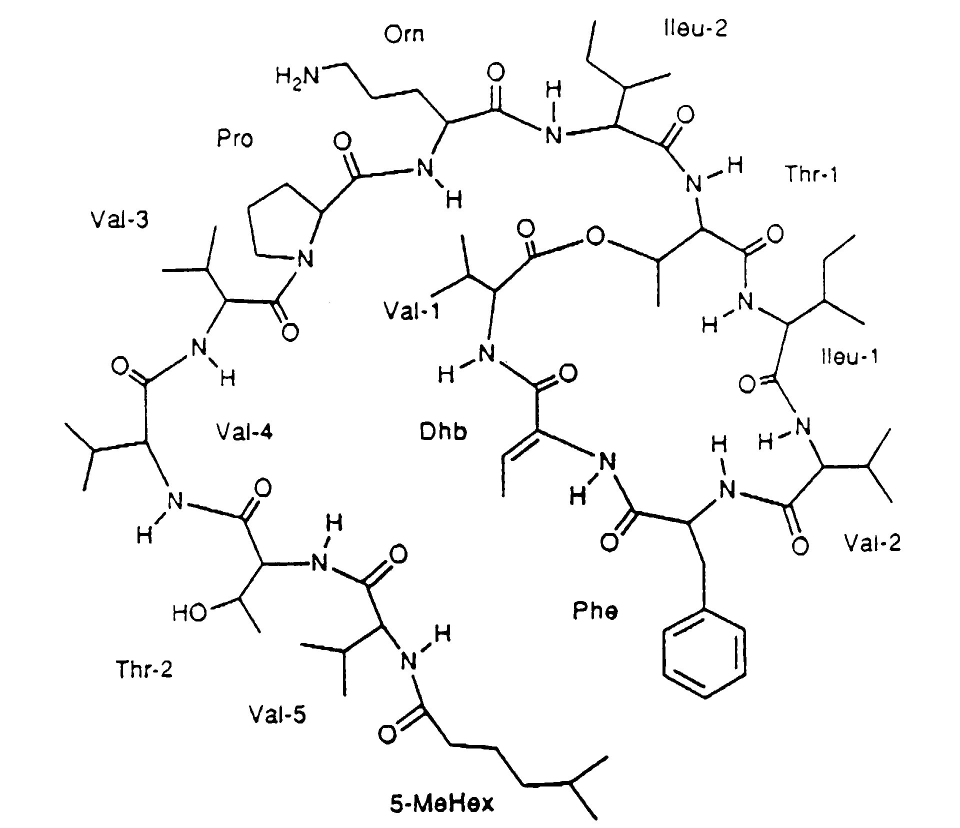

2. A pharmaceutical composition comprising Kahalalide F in association with a pharmaceutical carrier or diluent.

3. The use of Kahalalide F in the manufacture of an antitumor or antiviral pharmaceutical composition.

4. A process for the preparation of Kahalalide F of Claim 1, by extraction from the sacoglossan *Elysia rufescens.*

5. A process for preparing a pharmaceutical composition which comprises admixing a pharmaceutical carrier or diluent and Kahalalide F prepared by a process according to Claim 4.

6. A process according to Claim 5, wherein the pharmaceutical composition is for use in treating a mammal affected by a malignant tumor or for use in treating a viral infection.

## Patentansprüche

1. Kahalalid F mit der Formel:

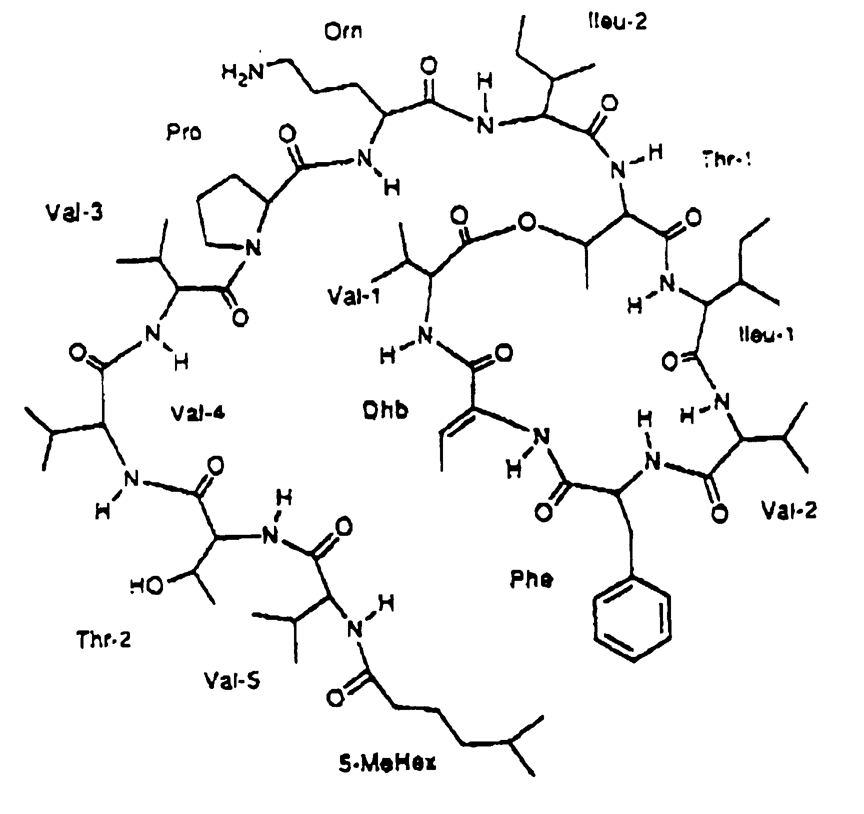

2. Pharmazeutische Zusammensetzung, die Kahalalid F in Assoziation mit einem pharmazeutischen Träger- oder Verdünnungsmittel enthält.

3. Einsatz von Kahalalid F bei der Herstellung einer pharmazeutischen Antitumor- oder Antivirus-Zusammensetzung.

4. Verfahren für die Herstellung von Kahalalid F nach Anspruch 1 durch Extraktion aus dem Sacoglossan *Elysia rufescens*.

5. Verfahren für die Zubereitung einer pharmazeutischen Zusammensetzung, welches das Beimischen eines pharmazeutischen Träger- oder Verdünnungsmittels zu Kahalalid F, das durch ein Verfahren nach Anspruch 4 hergestellt worden ist, umfaßt.

6. Verfahren nach Anspruch 5, bei dem die pharmazeutische Zusammensetzung für den Einsatz bei der Behandlung eines Säugers. der von einem malignen Tumor befallen ist, oder für die Behandlung einer Virusinfektion vorgesehen ist.

## Revendications

1. Kahalalide F de formule:

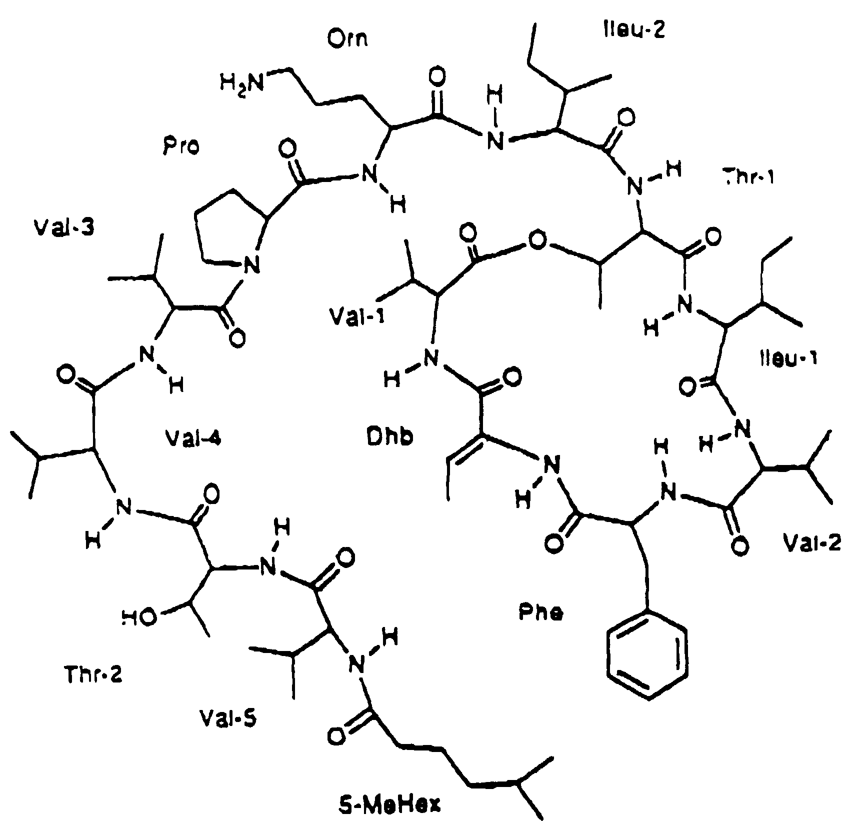

2. Composition pharmaceutique comprenant du Kahalalide F en association avec un véhicule ou un diluant pharmaceutique.

3. Utilisation de Kahalalide F dans la fabrication d'une composition pharmaceutique antitumorale ou antivirale.

4. Procédé pour la préparation du Kahalalide F de la revendication 1. par extraction du sacoglossan *Elysia rufescens.*

5. Procédé pour la préparation d'une composition pharmaceutique qui comprend le mélange d'un véhicule ou d'un diluant pharmaceutique et de Kahalalide F préparé par un procédé selon la revendication 4.

6. Procédé selon la revendication 5, dans lequel la composition pharmaceutique est destinée à être utilisée dans le traitement d'un mammifère atteint d'une tumeur maligne ou à être utilisée dans le traitement d'une infection virale.
